# EUROPÄISCHE PATENTSCHRIFT

(11) **EP 1 362 030 B1**
(45) Veröffentlichungstag und Bekanntmachung des Hinweises auf die Patenterteilung: **11.04.2012**
(21) Anmeldenummer: 02719831.6
(22) Anmeldetag: 14.02.2002
(51) Int. Cl.: C07D 207/34, A61K 31/40, A61P 9/10

(54) **NEUES BENZOYLGUANIDINSALZ**
NOVEL BENZOYLGUANIDINE SALT
SEL DE BENZOYLGUANIDINE

(30) Priorität: 15.02.2001 DE 10106970
(43) Veröffentlichungstag der Anmeldung: 19.11.2003
(73) Patentinhaber: Boehringer Ingelheim Pharma GmbH & Co.KG, 55216 Ingelheim am Rhein (DE)
(72) Erfinder: EICKMEIER, Christian, 88441 Mittelbiberach (DE); SIEGER, Peter, 88441 Mittelbiberach (DE); KÖRNER, Volkmar, 88440 Biberach (DE); HERTER, Rolf, 88400 Biberach (DE); RALL, Werner, 88441 Mittelbiberach (DE)
(86) Internationale Anmeldenummer: PCT/EP2002/001535
(87) Internationale Veröffentlichungsnummer: WO 2002/064563

(56) Entgegenhaltungen:
- WO-A-00/17176

## Beschreibung

Die Erfindung betrifft das Hydrochlorid des 4-[4-(2-Pyrrolylcarbonyl)-1-piperazinyl]-3-trifluormethyl-benzoylguanidins, Verfahren zu dessen Herstellung sowie dessen Verwendung zur Herstellung eines Arzneimittels.

### Hintergrund der Erfindung

Eine Reihe von Benzoylguanidin-Derivaten sind im Stand der Technik bekannt. So offenbart beispielsweise die International Patentanmeldung WO 00/17176 Benzoylguanidin-Derivate die durch wertvolle pharmakologische Eigenschaften gekennzeichnet sind. Diese Verbindungen wirken gegen Arrhythmien, die beispielsweise bei Hypoxien auftreten. Sie sind ferner anwendbar bei Krankheiten, die im Zusammenhang mit Ischämien stehen (Beispiele: cardiale, cerebrale, gastrointestinale - wie mensenteriale Thrombose/Embolie -, pulmonale, renale Ischämie, Ischämie der Leber, Ischämie der Skelettmuskulatur). Entsprechende Krankheiten sind beispielsweise coronare Herzerkrankung, Herzinfarkt, Angina pectoris, stabile Angina Pectoris, ventrikuläre Arrythmien, subventrikuläre Arrythmien, Herzinsuffizienz - ferner zur Unterstützung von Bypass-Operationen, zur Unterstützung von Operationen am offenen Herzen, zur Unterstützung von Operationen, die eine Unterbrechung der Blutversorgung des Herzens erforderlich machen und zur Unterstützung von Herztransplantationen - Embolie im Lungenkreislauf, akutes oder chronisches Nierenversagen, chronische Niereninsuffizienz, Himinfarkt, Reperfusionsschäden bei der Wiederdurchblutung von Himarealen nach Auflösung von Gefäßverschlüssen und akute und chronische Durchblutungsstörungen des Hirns. Hier sind die genannten Verbindungen auch in Kombination mit thrombolytischen Mitteln wie t-PA, Streptokinase und Urokinase nützlich.

Bei der Reperfusion des ischämischen Herzens (z. B. nach einem Angina-pectoris-Anfall oder einem Herzinfarkt) können irreversible Schädigungen an Cardiomyocyten in der betroffenen Region auftreten. Die Verbindungen wirken u. a. in einem solchen Fall cardioprotektiv.

In das Anwendungsgebiet ischämie ist auch die Verhinderung von Schäden an Transplantaten einzubeziehen (z. B. als Schutz des Transplantates -wie beispielseweise Leber, Niere, Herz oder Lunge - vor, während und nach der Implantation sowie bei der Lagerung der Transplantate), die im Zusammenhang mit Transplantationen auftreten können. Die in der WO 00/17176 offenbarten Verbindungen sind außerdem protektiv wirkende Arzneimittel bei der Durchführung angioplastischer operativer Eingriffe am Herzen und an peripheren Gefäßen.

Bei der essentiellen Hypertonie und diabetischen Nephropathie ist der zelluläre Natrium-Protonen-Austausch erhöht. Die Verbindungen eignen sich daher als Inhibitoren dieses Austausches zur vorbeugenden Behandlung dieser Krankheiten.

Die Verbindungen zeichnen sich weiterhin durch eine stark inhibierende Wirkung auf die Proliferation von Zellen aus. Deshalb sind die Verbindungen als Arzneimittel bei Krankheiten interessant, bei denen die Zellproliferation eine primäre oder sekundäre Rolle spielt und können als Mittel gegen Krebserkrankungen, gutartige Tumoren, oder beispielsweise Prostatahypertrophie, Atherosklesose, Organhypertrophien und - hyperplasien, fibrotische Erkrankungen und diabetische Spätkomplikationen verwendet werden.

Die vorstehend genannten pharmakologisch wertvollen Eigenschaften der im Stand der Technik offenbarten Benzoylguanidin-Derivate stellen die Grundvoraussetzung für eine wirksame Verwendung einer Verbindung als Arzneimittel dar. Ein Wirkstoff muß allerdings noch weiteren Anforderungen gerecht werden, um als Arzneimittel zum Einsatz gelangen zu können. Diese Parameter sind zu einem großen Teil mit der physico-chemischen Beschaffenheit des Wirkstoffs verbunden.

Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der Arzneimittelzusammensetzungen verwendete Arzneiwirkstoff sollte daher eine hohe Stabilität aufweisen, die auch unter verschiedenen Umgebungsbedingungen gewährleistet sein muß. Dies ist zwingend erforderlich, um zu verhindern, daß Arzneimittelzusammensetzungen Verwendung finden, in denen neben tatsächlichem Wirkstoff beispielsweise Abbauprodukte desselben enthalten sind. In einem solchen Fall könnte ein in pharmazeutischen Formulierungen vorgefundener Gehalt an Wirkstoff niedriger sein als spezifiziert.

Die Absorption von Feuchtigkeit vermindert den Gehalt an Arzneiwirkstoff wegen der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird. Vorzugsweise sollte ein Arzneimittelwirkstoff daher nur in geringem Maße hygroskopisch sein.

Da die Kristallmodifikation eines Wirkstoffs einen Einfluß auf die Wirksamkeit eines Arzneimittels haben kann, ist es notwendig, eventuell existierenden Polymorphismus eines kristallin vorliegenden Wirkstoffs bestmöglich aufzuklären. Sofern verschiedene polymorphe Modifikationen eines Wirkstoffs auftreten, sollte gewährleistet sein, daß sich die kristalline Modifikation der Substanz in der späteren Arzneimittelzubereitung nicht verändert. Andernfalls könnte dies die reproduzierbare Wirksamkeit des Medikaments nachteilig beeinflussen. Bevorzugt sind vor diesem Hintergrund Wirkstoffe, die nur durch geringen Polymorphismus gekennzeichnet sind.

Ein weiteres Kriterium, welches je nach Wahl der Formulierung oder nach Wahl des Herstellungsverfahrens der Formulierung von unter Umständen herausragender Bedeutung ist, ist die Löslichkeit des Wirkstoffs. Werden beispielsweise Arzneimittellösungen (beispielsweise für Infusionen) bereitgestellt, so ist eine ausreichende Löslichkeit des Wirkstoffs in physiologisch verträglichen Lösemitteln unverzichtbar. Auch für oral zu applizierende Arzneimittel ist eine ausreichende Löslichkeit des Wirkstoffs von großer Wichtigkeit.

Der vorliegenden Erfindung liegt die Aufgabe zugrunde, einen Arzneimittelwirkstoff bereitzustellen, der nicht nur durch eine hohe pharmakologische Wirksamkeit gekennzeichnet ist, sondern ferner den vorstehend genannten physico-chemischen Anforderungen bestmöglich gerecht wird.

### Detaillierte Beschreibung der Erfindung

Es wurde gefunden, daß die vorstehend genannte Aufgabe duch die Verbindung 4-[4-(2-Pyrrolylcarbonyl)-1-piperazinyl]-3-trifluormethyl-benzoylguanidin-hydrochlorid **1** 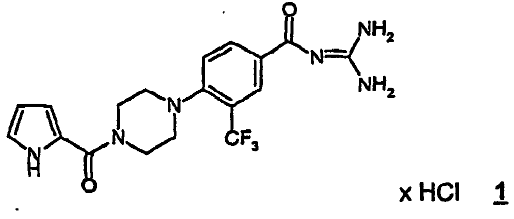 gelöst wird. Die Verbindung der Formel **1** ist nicht hygroskopisch und in physiologisch verträglichen Lösemitteln gut löslich. Sie ist ferner durch ein hohes Maß an Stabilität gekennzeichnet.

Das in der WO 00/17176 offenbarte Methansulfonat der Formel 1' 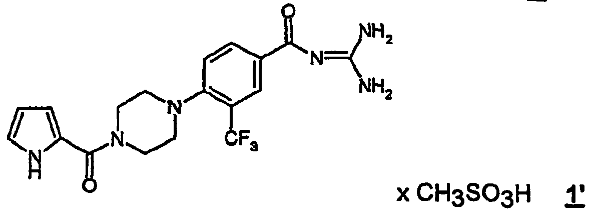 wird den eingangs erläuterten Anforderungen im Gegensatz zur Verbindung der Formel **1** dagegen nicht gerecht.

Dementsprechend betrifft ein Aspekt der vorliegenden Erfindung die Verbindung der Formel **1** als solche. Ein weiterer Aspekt der vorliegenden Erfindung betrifft die Verbindung der Formel **1** in Form ihrer Hydrate, bevorzugt in Form ihres Monohydrats oder ihres Halbhydrats.

Ein weiterer Aspekt der vorliegenden Erfindung betrifft die Verwendung der Verbindung der Formel **1** als Arzneimittel. Die vorliegende Erfindung betrifft ferner die Verwendung der Formel **1**, gegebenenfalls in Form ihrer Hydrate, zur Herstellung eines Arzneimittels zur Behandlung von Erkrankungen, in denen Inhibitoren des zellulären Na⁺/H⁺ -Austausches einen therapeutischen Nutzen entfalten können. Die vorliegende Erfindung betrifft ferner die Verwendung der Verbindung der Formel **1** zur Herstellung eines Arzneimittels zur Behandlung cardivaskulärer Erkrankungen. Die vorliegende Erfindung betrifft ferner die Verwendung der Verbindung der Formel **1** zur Herstellung eines Arzneimittels zur Behandlung von Arrhythmien, die beispielsweise bei Hypoxien auftreten. Die vorliegende Erfindung betrifft ferner die Verwendung der Verbindung der Formel **1** zur Herstellung eines Arzneimittels zur Behandlung von Krankheiten, die im Zusammenhang mit Ischämien stehen (Beispiele: cardiale, cerebrale, gastrointestinale -wie mensenteriale Thrombose/Embolie -, pulmonale, renale Ischämie, Ischämie der Leber, Ischämie der Skelettmuskulatur). Die vorliegende Erfindung betrifft ferner die Verwendung der Verbindung der Formel **1** zur Herstellung eines Arzneimittels zur Behandlung von Krankheiten die ausgewählt sind aus der Gruppe bestehend aus coronare Herzerkrankung, Herzinfarkt, Angina pectoris, stabile Angina Pectoris, ventrikuläre Arrythmien, subventrikuläre Arrythmien, Herzinsuffizienz - ferner zur Unterstützung von Bypass-Operationen, zur Unterstützung von Operationen am offenen Herzen, zur Unterstützung von Operationen, die eine Unterbrechung der Blutversorgung des Herzens erforderlich machen und zur Unterstützung von Herztransplantationen - Embolie im Lungenkreislauf, akutes oder chronisches Nierenversagen, chronische Niereninsuffizienz, Himinfarkt, Reperfusionsschäden bei der Wiederdurchblutung von Hirnarealen nach Auflösung von Gefäßverschlüssen und akute und chronische Durchblutungsstörungen des Hirns. Die vorliegende Erfindung betrifft ferner die Verwendung der Verbindung der Formel **1** zur Herstellung eines Arzneimittels zur Behandlung von Erkrankungen, in denen die Verwendung cardioprotektiver Wirkstoffe einen therapeutischen Nutzen entfalten kann. Die vorliegende Erfindung betrifft ferner die Verwendung der Verbindung der Formel **1** zur Herstellung eines Arzneimittels zur Behandlung von Krebserkrankungen, gutartigen Tumoren, oder beispielsweise Prostatahypertrophie, Atherosklesose, Organhypertrophien und - hyperplasien, fibrotische Erkrankungen und diabetischen Spätkomplikationen.

Die Verbindung der Formel **1** kann als wässerige injektionslösung (z.B. für intravenöse, intramuskuläre oder subkutane Applikation), als Tablette, als Suppositorium, als Salbe, als Pflaster für transdermale Applikation, als Aerosol für inhalative Anwendung über die Lunge oder als Nasenspray eingesetzt werden.

Der Wirkstoffgehalt einer Tablette oder eines Suppositoriums liegt zwischen 5 und 200 mg, vorzugsweise zwischen 10 und 50 mg. Bei inhalation liegt die Einzeldosis zwischen 0,05 und 20 mg, vorzugsweise zwischen 0,2 und 5 mg. Bei einer parenteralen Injektion liegt die Einzeldosis zwischen 0,1 und 50 mg, vorzugsweise zwischen 0,5 und 20 mg. Die genannten Dosen können, falls erforderlich, mehrmals täglich gegeben werden.

Im Folgenden sind einige Beispiele für pharmazeutische Präparate mit dem Wirkstoff angegeben:

### Tabletten:

| | |
|---|---|
| Verbindung der Formel **1** | 18,0 mg |
| Magnesiumstearat | 1,2 mg |
| Maisstärke | 60,0 mg |
| Lactose | 90,0 mg |
| Polyvinylpyrolidon | 1,5 mg |

### Lösung zur Infektion

| | |
|---|---|
| Verbindung der Formel **1** | 0,3 g |
| Natriumchlorid | 0,9 g |
| Aqua injectibilia | ad 100ml |

Diese Lösung kann unter Verwendung von Standardverfahren sterilisiert werden.

Die WO 00/17176 offenbart mögliche Herstellverfahren, die zur Synthese der freien Base 4-[4-(2-Pyrrolylcarbonyl)-1-piperazinyl]-3-trifluormethyl-benzoylguanidin zur Anwendung gelangen können. Ausgehend von dieser Verbindung werden nachfolgend mögliche synthetische Zugänge zur Verbindung der Formel **1** beispielhaft erläutert.

### Beispiel 1: 4-[4-(2-Pyrrolylcarbonyl)-1-piperazinyl]-3-trifluormethyl-benzoylguanidin-hydrochlorid

15,1 g 4-[4-(2-Pyrrolylcarbonyl)-1-piperazinyl]-3-trifluormethyl-benzoylguanidin werden in 151 ml Methanol aufgenommen und die erhaltene Suspension auf etwa 10°C abgekühlt. Zu dieser Suspension werden 16 ml einer gesättigten etherischen HCl-Lösung gegeben und damit auf pH 1 - 2 angesäuert. Unter Eiskühlung wird bis zur vollständigen Kristallisation nachgerührt. Die Kristalle werden abgesaugt, mit kaltem Methanol und anschließend mit kalterm Diethylether nachgewaschen.
Ausbeute: 16,19 g; Schmp.: 223 °C (unkorrigiert).

### Beispiel 2: 4-[4-(2-Pyrrolylcarbonyl)-1-piperazinyl]-3-trifluormethyl-benzoylguanidinhydrochlorid-hemihydrat

15,0 kg 4-[4-(2-Pyrrolylcarbonyl)-1-piperazinyl]-3-trifluormethyl-benzoylguanidin werden vorgelegt und mit 120 L Ethylacetat versetzt. Die Suspension wird auf etwa 45°C erwärmt und mit 30 L Wasser versetzt. Die erhaltene Mischung wird etwa 15 Minuten gerührt und die wässrige Phase anschließend abgetrennt. Zur organischen Phase wird bei konstanter Temperatur eine Lösung von 3,62 kg konzentrierter Salzsäure in 20 L Wasser gegeben. Innerhalb von etwa 1-2 Stunden wird auf 25°C -20°C abgekühlt. Das erhalten Hydrochlorid wird abgetrennt, mit 50 L Ethylacetat gewaschen und im Vakuum bei etwa 60°C getrocknet.
Ausbeute: 78 %; Schmp.: 225 ± 5 °C (DSC bei Heizrate 10K/min).

### Beispiel 3: 4-[4-(2-Pyrrolylcarbonyl)-1-piperazinyl]-3-trifluormethyl-benzoylguanidin-hydrochlorid-monohydrat

109,4 g 4-[4-(2-Pyrrolylcarbonyl)-1-piperazinyl]-3-trifluormethyl-benzoylguanidin werden in 1,5 L Wasser suspendiert und auf etwa 50°C erwärmt. 26,1 ml konzentrierte wässrige Salzsäure werden mit 300 ml Wasser verdünnt und innerhalb von etwa 20 Minuten zur vorgewärmten Suspension getropft. Es wird etwa 15 Minuten bei konstanter Temperatur nachgerührt. Anschließend wird die Temperatur unter Rühren über einen Zeitraum von etwa 1,5 Stunden auf etwa 35 °C abgesenkt. Danach wird auf 5 - 10 °C abgekühlt und eine weitere Stunde bei dieser Temperatur gerührt. Die erhaltenen Kristalle werden abgetrennt, mit wenig Wasser gewaschen und im Vakuum bei etwa 50 °C getrocknet.
Ausbeute: 116,5 g; Schmp.: 180 ± 5 °C (DSC bei Heizrate 10K/min).

## Patentansprüche

1. [4-(2-Pyrrolylcarbonyl)-1-piperazinyl]-3-trifluormethyl-benzoylguanidin-hydrochlorid **1**

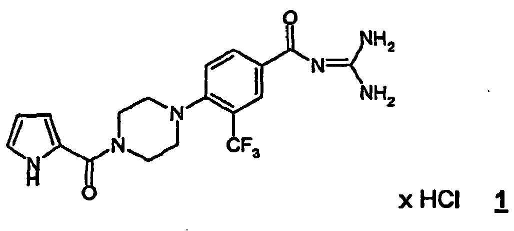

2. Verbindung nach Anspruch 1, **dadurch gekennzeichnet, daß** sie in Form eines ihrer Hydrate vorliegt.

3. Verbindung nach Anspruch 1 oder 2, **dadurch gekennzeichnet, daß** sie in Form ihres Monohydrats vorliegt.

4. Verbindung nach Anspruch 1 oder 2, **dadurch gekennzeichnet, daß** sie in Form ihres Hemihydrats vorliegt.

5. Verwendung der Verbindung der Formel **1** nach einem der Ansprüche **1** bis **4** als Arzneimittel.

6. Verwendung der Verbindung der Formel **1** nach einem der Ansprüche 1 bis 4 zur Herstellung eines Arzneimittels zur Behandlung von Erkrankungen, in denen Inhibitoren des zellulären Na⁺/H⁺-Austausches einen therapeutischen Nutzen entfalten können.

## Claims

1. [4-(2-pyrrolylcarbonyl)-1-piperazinyl]-3-trifluoromethyl-benzoylguanidine-hydrochloride **1**

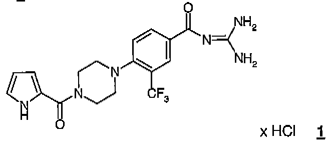

2. Compound according to claim 1, **characterised in that** it is present in the form of one of its hydrates.

3. Compound according to claim 1 or 2, **characterised in that** it is present in the form of its monohydrate.

4. Compound according to claim 1 or 2, **characterised in that** it is present in the form of its hemihydrate.

5. Use of the compound of formula **1** according to one of claims 1 to 4 as a medicament.

6. Use of the compound of formula 1 according to one of claims 1 to 4 in the preparation of a medicament for treating diseases in which inhibitors of the cellular Na⁺/H⁺ exchange may confer a therapeutic benefit.

## Revendications

1. Chlorhydrate de [4-(2-pyrrolylcarbonyl)-1-pipérazinyl]-3-trifluorométhyl-benzoylguanidine 1

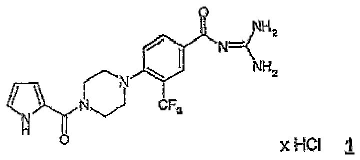

2. Composé selon la revendication 1, **caractérisé en ce qu'**il se présente sous la forme de l'un de ses hydrates.

3. Composé selon la revendication 1 ou 2, **caractérisé en ce qu'**il se présente sous la forme de son monohydrate.

4. Composé selon la revendication 1 ou 2, **caractérisé en ce qu'**il se présente sous la forme de son hémi-hydrate.

5. Utilisation du composé de formule 1 selon l'une des revendications 1 à 4 comme médicament.

6. Utilisation du composé de formule 1 selon l'une des revendications 1 à 4, pour la production d'un médicament destiné à traiter des maladies dans lesquelles des inhibiteurs de l'échange cellulaire de Na⁺/H⁺ peuvent déployer un bénéfice thérapeutique.
